(19) Europäisches Patentamt
European Patent Office
Office européen des brevets

(11) EP 1 062 195 B1

(12) **EUROPÄISCHE PATENTSCHRIFT**

(45) Veröffentlichungstag und Bekanntmachung des Hinweises auf die Patenterteilung:
**09.07.2003 Patentblatt 2003/28**

(21) Anmeldenummer: **99916818.0**

(22) Anmeldetag: **08.03.1999**

(51) Int Cl.[7]: **C07C 45/00**, C07C 47/04, C07D 323/06, C08G 2/08, C08G 65/00, C01B 3/22

(86) Internationale Anmeldenummer:
**PCT/EP99/01471**

(87) Internationale Veröffentlichungsnummer:
**WO 99/046228 (16.09.1999 Gazette 1999/37)**

(54) **VERFAHREN ZUR NICHT OXIDATIVEN HERSTELLUNG VON FORMALDEHYD AUS METHANOL**
METHOD FOR NON-OXIDATIVE PRODUCTION OF FORMALDEHYDE FROM METHANOL
PROCEDE DE PRODUCTION NON OXYDATIVE DE FORMALDEHYDE A PARTIR DE METHANOL

(84) Benannte Vertragsstaaten:
**BE DE FR GB NL**

(30) Priorität: **10.03.1998 DE 19810087**

(43) Veröffentlichungstag der Anmeldung:
**27.12.2000 Patentblatt 2000/52**

(73) Patentinhaber: **Ticona GmbH**
**65451 Kelsterbach (DE)**

(72) Erfinder:
- **KAISER, Thomas**
  **D-65779 Kelkheim (DE)**
- **SCHWEERS, Elke**
  **D-69812 Bad Soden (DE)**
- **MEISTER, Christine**
  **D-65843 Sulzbach (DE)**

(56) Entgegenhaltungen:
**EP-A- 0 294 684**
**EP-A- 0 691 338**
**DE-A- 1 645 451**
**DE-A- 19 644 188**
**DE-A- 19 814 281**
**DE-A- 19 814 283**
**DE-A- 19 814 284**
**DE-A- 19 814 285**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Mehrere Verfahren zur Herstellung von Formaldehyd aus Methanol sind bekannt (siehe z.B. Ullmann's Encyclopaedia of Industrial Chemistry). Technisch durchgeführt werden überwiegend die Oxidation

$$CH_3OH + ½\ O_2 \rightarrow CH_2O + H_2O$$

an Eisen- und Molybdänoxyd enthaltenden Katalysatoren bei 300°C bis 450°C (Formox Prozeß) und die oxidative Dehydrierung (Silberkontaktverfahren) gemäß:

$$CH_3OH \rightarrow CH_2O + H_2$$

$$H_2 + ½\ O_2 \rightarrow H_2O$$

bei 600°C bis 720°C. Nach beiden Verfahren liegt der Formaldehyd zunächst als wäßrige Lösung vor. Insbesondere bei der Verwendung für die Herstellung von Formaldehyd-Polymeren und -Oligomeren muß der so gewonnene Formaldehyd aufwendig entwässert werden. Ein weiterer Nachteil ist die Bildung korrosiver, die Polymerisation negativ beeinflussender Ameisensäure als Nebenprodukt.

Durch die Dehydrierung von Methanol können diese Nachteile vermieden und kann im Gegensatz zu oben genannten Verfahren nahezu wasserfreier Formaldehyd direkt gewonnen werden:

$$CH_3OH \overset{Kat.}{--->} CH_2O + H_2$$

Um ein ökologisches und wirtschaftlich interessantes technisches Verfahren für die Dehydrierung von Methanol zu erreichen, müssen die folgenden Voraussetzungen erfüllt werden: Die stark endotherme Reaktion muß bei hohen Temperaturen durchgeführt werden, um hohe Umsätze erreichen zu können. Konkurrierende Nebenreaktionen müssen unterdruckt werden, um eine hinreichende Selektivitat für Formaldehyd zu erzielen (unkatalysiert beträgt die Selektivität zu Formaldehyd unter 10 % bei Umsätzen über 90 %). Verweilzeiten müssen kurz bzw. die Abkühlung der Reaktionsprodukte schnell sein, um den Zerfall des thermodynamisch bei Reaktionsbedingungen nicht stabilen Formaldehyds gemäß

$$CH_2O \rightarrow CO + H_2$$

zu minimieren.

Verschiedene Verfahren zur Durchführung dieser Reaktion wurden vorgeschlagen; So ist beispielsweise in der DE-A-37 19 055 ein Verfahren zur Herstellung von Formaldehyd aus Methanol durch Dehydrierung in Gegenwart eines Katalysators bei erhöhter Temperatur beschrieben. Die Umsetzung wird in Gegenwart eines mindestens eine Natriumverbindung enthaltenden Katalysators bei einer Temperatur von 300°C bis 800°C durchgeführt.

J. Sauer und G. Emig (Chem. Eng. Technol. 1995,18, 284-291) gelang es, aus einem $NaAlO_2$ und $LiAlO_2$ enthaltendem Katalysator durch ein reduzierendes Gasgemisch (87% $N_2$ + 13% $H_2$) eine katalytisch aktive Spezies freizusetzen, bei der es sich seiner Vermutung nach um Natrium handele. Diese Spezies vermag die Dehydrierung von im gleichen Reaktor stromabwärts zugegebenem, d.h. nicht mit der Katalysatorschüttung in Kontakt gekommenem, Methanol zu Formaldehyd zu katalysieren. Bei Verwendung nicht reduzierender Gase wurde nur eine geringe katalytische Aktivität beobachtet.

Nach J. Sauer und G. Emig sowie Ergebnissen aus neueren Untersuchungen (siehe z.B. M. Bender et al., Vortrag auf dem XXX. Jahrestreffen deutscher Katalytiker, 21.-23.3.1997) wurden Natriumatome und NaO-Moleküle als in die Gasphase emittierte Verbindungen identifiziert und deren katalytische Aktivität für die Dehydrierung von Methanol in der Gasphase beschrieben. Das Ausgangsmaterial Methanol wird bei den bekannten Verfahren stets verdünnt mit Stickstoff und/oder Stickstoff/Wasserstoff-Gemischen umgesetzt In verschiedenen Schriften, wie EP-A 0 130 068, EP-A 0 261 867 und DE-A 25 25 174 wird vorgeschlagen, das bei der Reaktion entstehende Gasgemisch nach Abtrennen des Formaldehyds als Brennstoff zu verwenden.

Obwohl mit den bekannten Verfahren bereits gute Ergebnisse erzielt werden, besteht doch ein breiter Raum für Verbesserungen in technischer und ökonomischer Hinsicht.

Der Erfindung lag daher die Aufgabe zugrunde, ein verbessertes und wirtschaftlicheres Verfahren bereitzustellen.

Auf der Suche nach der Lösung dieser Aufgabe wurde folgendes gefunden:

Eine in technischer und wirtschaftlicher, insbesondere energetischer Hinsicht stark verbesserte Reaktionsführung läßt sich erreichen, wenn das neben dem Formaldehyd entstehende Produktgasgemisch zur Verdünnung des Ausgangsstoffes Methanol eingesetzt wird.

Weiterhin vorteilhaft ist die Erzeugung einer katalytisch aktiven Spezies aus einem Primärkatalysator in einem Trägergasstrom bei einer Temperatur, die nicht gleich der Dehydrierungstemperatur ist. Die Bereiche zur Erzeugung der katalytisch aktiven Spezies und des Reaktionsteils sind vorteilhafterweise räumlich voneinander getrennt. Dadurch können unterschiedliche Temperaturen und Verweilzeiten für die durch diese Einheiten geleiteten Trägergasströme eingestellt werden.

Der Kreisgasstrom wird erhalten, indem man nach Abtrennung des Formaldehyds die Nebenprodukte der Dehydrierung, vornehmlich $H_2$ und CO, mittels einer geeigneten Vorrichtung zumindest teilweise in den Reaktor zurückführt.

Aus geeigneten Primärkatalysatoren wird bei thermischer Behandlung in der Primärkatalysatorzersetzungszone und beim Überströmen mit Gas bei Temperaturen, die nicht gleich der Dehydrierungstemperatur sind, eine oder mehrere katalytisch aktive Spezies ausgetragen oder erzeugt, die in der Lage sind, die Dehydrierung von Methanol zu katalysieren. Ein solch fluider Katalysator wird über die Zuleitungen in die Reaktionszone transportiert. Diese getrennte Temperatureinstellung erlaubt durch Anpassung an die jeweiligen Bedingungen zur Katalysatonreisetzung/-verdampfung bzw. Erzeugung einer katalytisch aktiven Spezies einerseits und zur Reaktion andererseits insbesondere die Möglichkeit zur Erniedrigung der Reaktionstemperatur. Damit vermindert sich der Zerfall des instabilen Formaldehyds durch Folgereaktionen und erhöht sich die Ausbeute.

Obwohl in der Literatur die homogen katalysierte nicht oxidative Dehydrierung von Methanol mit Natriumspezies in der Gasphase und die Zuführung der katalytisch aktiven Spezies durch thermische Zersetzung eines Primärkatalysators oder Verdampfung verschiedener Substanzen beschrieben ist, wurden bisher jedoch noch nicht mögliche Verluste des Katalysators auf dem Weg zur Reaktionszone beachtet.

Überraschend zeigte sich, daß die Katalysatorausnutzung und Bildung von Ablagerungen im Bereich der Erzeugung der aktiven Katalysatorspezies stark von den eingesetzten Trägergasen abhängt.

Bei Verwendung von $H_2$/CO-Gemischen oder des Kreisgases als Trägergas für die Aufnahme der Katalysatorspezies bilden sich in der Zuleitung vor dem eigentlichen Reaktor aufwachsende Ablagerungen, die hauptsächlich Kohlenstoff und daneben Natrium, Sauerstoff und Wasserstoff enthalten. Dadurch kommt es zu einer Verschlechterung der Primärkatalysatorausnutzung sowie teilweisen Inaktivierung des Katalysators und einer Begrenzung der störungsfreien Betriebsdauer der Anlage. Dieser negative Synergieeffekt aus Bildung einer Ablagerung und Einlagerung des Katalysators verschlechtert den ökonomischen Betrieb der Anlage.

Es wurde weiter überraschend gefunden daß es möglich ist. die Primärkatalysatorausnutzung zu erhöhen und die Bildung von Ablagerungen im Bereich der Katalysatorzuführung und Reaktoreingang zu vermeiden.

Gegenstand der Erfindung ist daher ein Verfahren zur Herstellung von Formaldehyd aus Methanol durch Dehydrierung in einem Reaktor in Gegenwart eines Katalysators, den man mit Hilfe eines Trägergases in den Reaktor einträgt, bei Temperaturen aus dem Bereich von 300 bis 1000°C, wobei ein Produktgasgemisch entsteht, dadurch gekennzeichnet, daß man aus dem Produktgasgemisch den Formaldehyd abtrennt und das verbleibende Produktgasgemisch mindestens teilweise in den Reaktor in einem Kreisgasstrom zurückführt und daß man als Trägergas ein kohlenstofffreies Gas oder Gasgemisch, insbesondere Stickstoff, Wasserstoff, ein Edelgas oder eine Mischung aus diesen Gasen, verwendet.

Besondere Ausführungsformen sind in den Unteransprüchen offenbart. Es können auch einzelne oder mehrere dieser Ausführungsformen jeweils für sich oder in Kombination Lösungen der Aufgabe darstellen und es sind auch die Merkmale der Ausführungsformen beliebig kombinierbar.

Der Anteil des katalysatorhaltigen Trägergasstroms zum Gesamtstrom beträgt hierbei 1 bis 60%, insbesondere 1 bis 50%, besonders bevorzugt 5 bis 40%.

Das erfindungsgemäße Verfahren zeichnet sich durch eine ökologisch und ökonomisch günstige Gewinnung von wasserarmem Formaldehyd aus. Durch die Nutzung der wasserstoffreichen Nebenprodukte der Reaktion, d.h. des Produktgases nach Abtrennen des Formaldehyds, zur Verdünnung des Edukts Methanol für die Dehydrierung lassen sich einerseits besonders hohe Ausbeuten erreichen und andererseits wegen der guten Wärmeleitfähigkeit der apparative Aufwand für die Erwärmung der Edukte, den Eintrag der Reaktionswärme sowie die Abkühlung der Produkte minimieren. Durch die weitere mögliche Nutzung von Teilen der Nebenprodukte der Reaktion, d.h. des Produktgases

nach Abtrennen des Formaldehyds, als Brennstoff zur Erzeugung der nötigen Reaktionstemperatur für die Dehydrierung, sowie durch eine Wärmerückgewinnung aus den Abgasen kann die Wärme für diesen und weitere Prozeßschritte gewonnen werden. Den Prozeß verlassen dann im wesentlichen nur der Wertstoff Formaldehyd und die Verbrennungsprodukte $CO_2$ und $H_2O$.

Durch das erfindungsgemäße Verfahren lassen sich weiterhin Verluste des Katalysators auf dem Weg zur Reaktionszone und Betriebsstörungen der Anlage durch die sich bildenden Ablagerungen vermeiden. Dies verringert zudem die Notwendigkeit der aus ökonomischer und ökologischer Sicht unerwünschten Betriebsunterbrechungen und Reinigungsschritte.

Vorteile der tieferen Reaktionstemperatur liegen in dem geringeren energetischen und apparativen Aufwand zur Erwärmung/Abkühlung vor/nach der Reaktion, der geringen Zerfallsgeschwindigkeit des bei Reaktionsbedingungen thermisch instabilen Formaldehyds und den geringen Anforderungen an die Werkstoffe.

Dehydrierung im Sinne der Erfindung bedeutet einen nicht oxidativen Prozeß gemäß der Gleichung:

$$CH_3OH \overset{\text{Kat.}}{\text{--->}} CH_2O + H_2$$

Das Kreisgas im Sinne der Erfindung bezeichnet das nach Abtrennen des Produkts Formaldehyd verbleibende Produktgasgemisch. welches neben Wasserstoff üblicherweise CO, $CH_4$, und $CO_2$ sowie gegebenenfalls $CH_2O$, MeOH, $H_2O$, $HCOOCH_3$ enthält und vorzugsweise im wesentlichen aus $H_2$, CO, $CH_4$ und $CO_2$ besteht. Besonders bevorzugt ist das Verhältnis $H_2/CO$ im Kreisgas $\geq 3$.

Figur 1 gibt in Form eines schematischen Verfahrensfließbildes einen Überblick über eine bevorzugte Variante des erfindungsgemäßen Verfahrens.

Aus einem Vorratsgefäß 1 wird Methanol 2 in einem Wärmeaustauscher 3 vorgewärmt und verdampft und nach Verdünnung mit in einem Wärmeaustauscher 18 vorgewärmten Kreisgas 19 in den Reaktor 4 geleitet.

Aus einem Vorratsbehälter 5 wird Primärkatalysator 6 in den beheizten Behälter 7 gefordert, der mit einem Trägergas 8, das durch den Warmetauscher 9 erwärmt wird durchströmt wird Dieser Strom wird durch eine Leitung 10 ebenfalls in den Reaktor 4 geleitet. Nach Durchlaufen des Reaktors 4 wird in einem Wärmetauscher 11 abgekühlt und das Produktgasgemisch 12 im Separationsgefäß 13 in Formaldehyd 14 und Nebenprodukte 15 (Kreisgas) aufgetrennt. Die Nebenprodukte werden mittels einer Fördereinrichtung 16, beispielsweise einem Ventilator, zumindest teilweise im Kreis in den Reaktor zurückgeführt. Ein Teil der Nebenprodukte kann nach Ausschleusung direkt als Brennmaterial in einer Vorrichtung zur Befeuerung des Reaktionsgefäßes 17 verwendet werden.

Das erfindungsgemäße Verfahren kann in einer Vorrichtung enthaltend einen oder mehrere Wärmeaustauscher zum Vorwärmen der Ausgangsstoffe Methanol und Kreisgas, eine Einheit zur Freisetzung des Katalysators in einen Trägergasteilstrom, einen Reaktor zur Durchführung der Dehydrierung, einen Wärmeaustauscher zum Abkühlen des Produktgasgemisches, ein Separationsgefäß zum Abtrennen des Formaldehyds, sowie Mittel, insbesondere ein Ventilator, zur Rückführung zumindest eines Teils der Nebenprodukte der Reaktion in den Reaktor, durchgeführt werden.

In einer bevorzugten Ausführungsform enthält die Vorrichtung weiterhin Mittel zur Ausschleusung eines weiteren Teils der Nebenprodukte der Dehydrierung, durch die dieser Teil einer Vorrichtung zur Beheizung des Reaktors zugeführt wird und dort als Brennmaterial dient.

Für die Reaktion kann handelsübliches Methanol eingesetzt werden, vorzugsweise sollte es wasserarm sein und keine Stoffe enthalten, die den Katalysator vergiften.

Zur Durchführung der Dehydrierung wird das fluide, vorzugsweise gasförmige Methanol mit gasförmigen Nebenprodukten der Dehydrierung verdünnt.

Der molare Methanolanteil beträgt im allgemeinen 5 bis 90 %, vorzugsweise 10 bis 60 %, besonders bevorzugt 10 bis 50 %. Aus dem Methanolanteil folgt die Menge des benötigten Kreisgases.

Als Primärkatalysatoren werden im erfindungsgemäßen Verfahren bevorzugt natriumhaltige Verbindungen, besonders bevorzugt metallisches Natrium und Natriumalkoholate niederer ($C_1$-$C_6$) Alkohole eingesetzt.

Die obengenannten Verbindungen liefern als Katalysatoren in einer Figur 1 entsprechenden Anlage Formaldehyd in Ausbeuten von über 70% und geringen Wasserkonzentrationen von weniger als 5 mol-% $H_2O$ pro mol Formaldehyd.

Die Nachführung des Primärkatalysators als Feststoff, z.B. pulverförmig, körnig oder kompaktiert, erfolgt über eine Feststoffdosierung, z.B. mit Hub- oder Drehkolben, Zellradschleuse, Schnecke oder Schüttelrinne.

Wird der Primärkatalysator gelöst zugegeben, sind besonders Lösungsmittel mit einer chemischen Zusammensetzung geeignet, die nur die im Prozeß schon vorhandenen Elemente (C, H, O) enthalten und somit nicht stören. Besonders bevorzugt wird MeOH als Lösungsmittel. Die Zugabe erfolgt z.B. über eine Düse, die gekühlt werden kann,

um ein Verdampfen des Lösungsmittels, Auskristallisieren oder Ablagerungen des festen Primärkatalysators in der Düse zu vermeiden.

Die Zugabe des Primärkatalysators als Schmelze ist z.B. über eine Düse möglich. Die Schmelze kann dann direkt im Gasstrom verdampft oder zersetzt werden.

Dies kann beispielsweise durch Aufbringen des Katalysatormaterials nach den oben beschriebenen Verfahren auf eine geeignete Oberfläche erreicht werden, die vom Gas durch- oder überströmt wird. Es kann sich hierbei um die Oberfläche eines Trägermaterials handeln, das in einem Festbett als Schüttung angeordnet ist. Als Materialien eignen sich z.B. SiC, $SiO_2$, $Al_2O_3$ etc. in einer geeigneten geometrischen Form, z.B. als Granulat, Pellets oder Kugeln. Die Anordnung geschieht vorzugsweise senkrecht in einem Festbeit, vorzugsweise mit Dosierung von oben. Die eingetragene Substanz schlägt sich auf dem Trägermaterial nieder und die katalytisch aktive Substanz geht während des Prozesses in die Gasphase über.

Eine andere Möglichkeit ist die Anordnung des Primärkatalysators in eine Wirbelschicht, durch die der Trägergasstrom geleitet wird. Das Wirbelgut besteht dabei zumindest teilweise aus dem geträgerten oder ungeträgerten Primärkatalysator. Der Verlust an aktiver Substanz kann durch Nachführung von frischem Primärkatalysator ersetzt werden, verbrauchtes Material kann gegebenenfalls abgezogen werden. Realisiert werden kann dies im kontinuierlichen Fall z.B. durch eine zirkulierende Wirbelschicht.

Die Nachführung eines Primärkatalysators kann auch durch abwechselnde Sekundärkatalysatorerzeugung in verschiedenen Behältern erfolgen, in denen der Primärkatalysator beispielsweise als Festbett oder Wirbelschicht, jeweils geträgert oder ungeträgert, angeordnet sein kann.

Der Vorteil der Verwendung mehrerer Einheiten zur diskontinuierlichen Katalysatornachführung besteht darin, daß auch solche Primärkatalysatoren eingesetzt werden, bei denen, z.B. aufgrund von Stoffeigenschaften wie z.B. Schmelzpunkt, Viskosität oder Zersetzungstemperatur, eine kontinuierliche Förderung nicht oder nur mit großem Aufwand möglich wäre.

Geeignete Reaktoren sind dem Fachmann bekannt und geläufig. Grundsätzlich können Reaktortypen und Aufbauten verwendet werden, wie sie aus der Literatur für Dehydrierungsreaktionen bekannt sind. Solche Apparaturen sind beispielsweise in Winnacker/Küchler, Chemische Technologie, 4. Auflage, Kapitel "Technik der Pyrolyse", Hanser Verlag, München 1981-86, beschrieben.

Geeignet sind beispielsweise Rohrreaktoren; geeignete Reaktormaterialien sind beispielsweise keramische Werkstoffe, wie Korund, aber auch aufkohlungs-, temperatur- und zunderbeständige Eisen- und Nickelbasislegierungen, wie Inconel 600® oder Hasteloy®.

Die dem Prozeß zuzuführende Wärme wird vorzugsweise durch die Verbrennung von aus dem Kreisprozeß ausgeschleusten Nebenprodukten der Dehydrierung, vornehmlich $H_2$ und CO, gewonnen.

Wird der Reaktor durch eine Verbrennungsreaktion beheizt, eignet sich z.B. ein von außen befeuerter Rohrreaktor.

Ebenfalls bevorzugt ist die Beheizung des Reaktors durch Mikrowellen.

Die Abtrennung des Formaldehyds aus dem Reaktionsgemisch kann nach an sich bekannten, dem Fachmann geläufigen Methoden erfolgen, beispielsweise durch Kondensation, Polymerisation oder physikalische oder chemische Ab- oder Adsorption.

Eine technisch erprobte Methode ist die Bildung von Halbacetalen aus Formaldehyd und einem Alkohol. Die Halbacetale werden daran anschließend thermisch gespalten, wobei sehr reiner Formaldehyddampf entsteht. Als Alkohol wird meist Cyclohexanol verwendet, da dessen Siedepunkt genügend weit über der Zersetzungstemperatur des Halbacetals liegt. Die Halbacetale werden üblicherweise in Fallfilm- oder Dünnschichtverdampfem bei Temperaturen von 100 bis 160°C gespalten (siehe z.B. US 2,848,500 vom 19.08.1958 "Preparation of Purified Formaldehyde" und US 2,943,701 vom 05.07.1960 "Process for purification of gaseous formaldehyde", oder JP-A 62/289 540). Die dabei frei werdenden Formaldehyddämpfe enthalten noch geringe Mengen Verunreinigungen, die meist durch eine Gegenstromwäsche mit Alkohol, wie Cyclohexanolhemiformal, durch Kondensation oder auch durch gezielte Präpolymerisation, entfernt werden.

Eine weitere Methode zur Abtrennung von Formaldehyd aus dem Reaktionsgemisch ist die Bildung von Trioxan in einem katalytischen Gasphasenprozeß (siehe z.B. Appl Catalysis A 1997 150. 143-151 und EP-A 0 691 338) Trioxan kann dann z B. auskondensiert werden.

Verwertungsmöglichkeiten für die Nebenprodukte der Reaktion, insbesondere Wasserstoff, sind beispielsweise die Synthese von Methanol oder die Gewinnung von reinem Wasserstoff, der z.B. durch Membranen abgetrennt werden kann.

So gewonnener Wasserstoff eignet sich beispielsweise zur Synthese von Ammoniak, in Raffinerieprozessen zur Herstellung von Benzin und Crackprodukten der Petrochemie, zur Methanolsynthese, zur Fetthärtung u.a. Hydrierungen, als Reduktionsmittel zur Gewinnung von W, Mo, Co u.a. Metallen, als reduzierendes Schutzgas bei metallurgischen Prozessen, zu autogenem Schweißen und Schneiden, als Brenngas in Mischung mit anderen Gasen (Stadtgas, Wassergas), oder verflüssigt als Treibstoff in Luft- und Raumfahrt.

Der nach dem erfindungsgemäßen Verfahren hergestellte Formaldehyd eignet sich für alle bekannten Einsatzgebiete, beispielsweise Korrosionsschutz, Spiegelherstellung, elektrochemische Beschichtungen, zur Herstellung von methanolischen Formaldehydlösungen und Methylal, zur Desinfektion und als Konservierungsmittel, ebenso als Zwischenprodukt zur Herstellung von Kunststoffen, beispielsweise, Polyacetalen (Polyoxymethylenen), Phenolharzen, Melaminen, Aminoplasten, Polyurethanen und Caseinkunststoffen, 1,4-Butanole, Trlmethylolpropan, Neopentylglykol, Pentaerythrit und Trioxan, zur Herstellung von Farbstoffen, wie Fuchsin, Acridin, zur Herstellung von Düngemitteln sowie zur Behandlung von Saatgut.

Da Formaldehyd nach dem erfindungsgemäßen Verfahren üblicherweise mit geringem Wassergehält hergestellt wird, eignet sich so hergestellter Formaldehyd insbesondere für die Polymerisation zu Polyoxymethylen und Trioxan, da hier wasserfreier Formaldehyd einzusetzen ist. Die Erfindung betrifft auch ein Verfahren zur Herstellen von Polyoxymethylen, und Polyacetalen und Trioxan.

Die Erfindung wird durch die Beispiele näher erläutert, ohne dadurch jedoch eingeschränkt zu werden.

Beispiele

Die angegebenen Meßgrößen werden wie folgt berechnet:

$$\text{Ausbeute (in \%)} = \frac{\text{gebildeter Formaldehyd (mol)}}{\text{zudosiertes Methanol (mol)}} \cdot 100\ \%$$

A Elektrisch beheizter Rohreaktor:

Die Dehydrierung des Methanols wird in einem Rohrreaktor 4, der indirekt durch einen elektrischen Rohrofen beheizt wird, durchgeführt (Aufbau der Versuchsapparatur gemäß Fließbild in Figur 2). Die Erzeugung des katalysatorhaltigen Stromes erfolgt durch Überleiten eines Trägergasstromes 8 (Stickstoff, Wasserstoff oder Argon und/oder $H_2$/CO bzw. Kreisgas) über ein Schiffchen mit Primärkatalysator 6 (metallisches Natrium oder Natriummethylat) das sich in einem beheizten Metallrohr 7 befindet. Das Methylat wird zersetzt bzw. das Natrium verdampft. Der Strom wird durch eine beheizte Transferleitung 10, deren Temperatur höher als die im Metallrohr der Katalysatorzersetzung 7 ist, in den Reaktionsraum geleitet.

Methanol 2 wird mit einer Pumpe gefördert und mit einem Wärmetauscher 3 vorgewärmt oder verdampft, in den Kreisgasstrom 19 geleitet, weiter auf eine Temperatur nahe der Reaktionstemperatur erwärmt und ebenfalls dem Reaktionsraum 4 zugegeben. Der Reaktionsraum wird durch ein Rohr mit der Länge 300-450 mm, Innendurchmesser 11 mm gebildet. Die Reaktortemperatur beträgt 600-850°C. Der Gesamtstrom beträgt 100-500 l/h. Die Methanolzufuhr ist so bemessen, daß sich eine Methanolkonzentration von ca. 10-20 mol-% einstellt.

Die Produktgase 12 werden in einem Kühler 11 nach Austritt aus dem Reaktor rasch auf eine Temperatur kleiner 200°C gekühlt und mittels eines Gaschromatographen analysiert. In einer Kolonne 13 werden die Reaktionsprodukte mit Alkohol (z.B. Cyclohexanol bei 20-80°C) gewaschen, um den Formaldehyd auszutragen. Die gasförmigen Nebenprodukte werden mit einem Ventilator 16 zurückgeführt, ein Überschuß 17 ausgeschleust.

Zur Beurteilung der Katalysatorausnutzung und Bildung von Ablagerungen wurde nach jedem Versuch die den Katalysator führenden Zuleitungen mechanisch gereinigt und gespült und die Menge der Ablagerungen bestimmt und gegebenenfalls analysiert.

Versuchsbedingungen:

Anfahren der Anlage vor Umschalten auf Kreisgas mit $H_2$/CO (ca. 15% CO) Kreisgasstrom aus Produktgasgemisch nach Wäsche bzw. $H_2$/CO (ca. 15% CO) 200-350 l/h Trägergasgesamtstrom durch Reaktor,
20-100 l/h Trägergasstrom durch Katalysatorfreisetzung,
42-56 g/h Methanol,
eingestellte Temperaturen:

Ofentemperatur Katalysatorfreisetzung 300-700°C, Transferleitung 500-700°C, Ofentemperatur Reaktor 750-900°C.

Tabelle 1:

| Formaldehydausbeute und Bildung von natriumhaltigen Ablagerungen bei der Dehydrierung von Methanol | | | | |
|---|---|---|---|---|
| Beispiel Vergleichsbeispiel | Katalysator | Trägergas Katalysator | Ausbeute Formaldehyd | Ablagerungen in Katalysator-transferleitung |
| Bsp. 1 | Na | $N_2$ | 73 | keine |
| Bsp. 2 | Na | $N_2$ | 70 | keine |
| Bsp. 3 | Na-methylat | $N_2$ | 69 | keine |
| Bsp. 4 | Na | $H_2$ | 65 | keine |
| Bsp. 5 | Na | Ar | 68 | keine |
| Bsp. 6 | Na-methylat | Ar | 65 | keine |
| Gegenbsp 1 | Na | $H_2$/CO | 68 | Zuleitung nach 3 h verstopft, Ablagerung aus C, Na, H und O |
| Gegenbsp. 2 | Na-methylat | $H_2$/CO | 65 | Zuleitung nach 5 h verstopft, Ablagerung aus C, Na, H und O |

**Patentansprüche**

1. Verfahren zur Herstellung von Formaldehyd aus Methanol durch Dehydrierung in einem Reaktor in Gegenwart eines Katalysators, den man mit Hilfe eines Trägergases in den Reaktor einträgt, bei Temperaturen aus dem Bereich von 300 bis 1000°C wobei ein Produktgasgemisch entsteht, **dadurch gekennzeichnet, daß** man aus dem Produktgasgemisch den Formaldehyd abtrennt und das verbleibende Produktgasgemisch mindestens teilweise in den Reaktor in einem Kreisgasstrom zurückführt und daß man als Trägergas ein kohlenstofffreies Gas oder Gasgemisch verwendet.

2. Verfahren gemäß Anspruch 1, **dadurch gekennzeichnet, daß** das kohlenstofffreie Gas Stickstoff oder Wasserstoff oder ein Edelgas oder eine Mischung aus diesen Gasen ist.

3. Verfahren gemäß Anspruch 1 oder 2, **dadurch gekennzeichnet, daß** der Anteil an kohlenstofffreiem Trägergas durch die Katalysalorzuführung vom Gesamtstrom 1 bis 50% beträgt.

4. Verfahren gemäß einem oder mehreren der Ansprüche 1 bis 3, **dadurch gekennzeichnet, daß** man als Katalysator Natrium einsetzt.

5. Verfahren gemäß einem oder mehreren der Ansprüche 1 bis 4, **dadurch gekennzeichnet, daß** man als Katalysator Natriumalkoholate niederer ($C_1$-$C_6$) Alkohole einsetzt.

6. Verfahren gemäß einem oder mehreren der Ansprüche 1 bis 5, **dadurch gekennzeichnet, daß** als Reaktor ein von außen befeuerter Rohrreaktor eingesetzt wird.

7. Verfahren gemäß einem oder mehreren der Ansprüche 1 bis 6, **dadurch gekennzeichnet, daß** ein Teil der Nebenprodukte der Dehydrierung zur Energiegewinnung bzw. als Brennstoff zur Beheizung des Reaktors verwendet wird.

8. Verfahren gemäß einem oder mehreren der Ansprüche 1 bis 7, **dadurch gekennzeichnet, daß** man den als Nebenprodukt anfallenden Wasserstoff abtrennt und einer Verwertung zuführt.

9. Verfahren zur Herstellung von Trioxan, **dadurch gekennzeichnet, daß** man

   - Formaldehyd aus Methanol durch Dehydrierung in einem Reaktor in Gegenwart eines Katalysators, den man mit Hilfe eines Trägergases in den Reaktor einträgt, bei Temperaturen aus dem Bereich von 300 bis 1000°C herstellt, wobei ein Produktgasgemisch entsteht, aus dem man den Formaldehyd abtrennt und das verbleibende Produktgasgemisch mindestens teilweise in den Reaktor in einem Kreisgasstrom zurückführt und man als Trägergas ein kohlenstoffreies Gas oder Gasgemisch verwendet und

- den so gewonnenen Formaldehyd zu Trioxan trimerisiert.

**10.** Verfahren zur Herstellung von Polyoxymethylen, **dadurch gekennzeichnet, daß** man

- Formaldehyd aus Methanol durch Dehydrierung in einem Reaktor in Gegenwart eines Katalysators, den man mit Hilfe eines Trägergases in den Reaktor einträgt, bei Temperaturen aus dem Bereich von 300 bis 1000°C herstellt, wobei ein Produktgasgemisch entsteht, aus dem man den Formaldehyd abtrennt und das verbleibende Produktgasgemisch mindestens teilweise in den Reaktor in einem Kreisgasstrom zurückführt und man als Trägergas ein kohlenstofffreies Gas oder Gasgemisch verwendet und
- den so gewonnenen Formaldehyd gegebenenfalls reinigt, and
- den Formaldehyd polymerisiert.

## Claims

**1.** A process for preparing formaldehyde from methanol by dehydrogenation in a reactor at temperatures in the range from 300 to 1000°C in the presence of a catalyst which is introduced into the reactor with the aid of a carrier gas to give a product gas mixture, wherein the formaldehyde is separated from the product gas mixture and at least part of the remaining product gas mixture is recirculated to the reactor in a circulating gas stream and the carrier gas used is a carbon-free gas or gas mixture.

**2.** The process as claimed in claim 1, wherein the carbon-free gas is nitrogen or hydrogen or a noble gas or a mixture of these gases.

**3.** The process as claimed in claim 1 or 2, wherein the amount of carbon-free carrier gas via the catalyst introduction makes up from 1 to 50% of the total gas stream.

**4.** The process as claimed in one or more of claims 1 to 3, wherein the catalyst used is sodium.

**5.** The process as claimed in one or more of claims 1 to 4, wherein the catalyst used is a sodium alkoxide of a lower ($C_1$-$C_6$) alcohol.

**6.** The process as claimed in one or more of claims 1 to 5, wherein the reactor used is an externally fired tube reactor.

**7.** The process as claimed in one or more of claims 1 to 6, wherein part of the by-products of the dehydrogenation is used for energy recovery or as fuel for heating the reactor.

**8.** The process as claimed in one or more of claims 1 to 7, wherein the hydrogen formed as by-product is separated off and used elsewhere.

**9.** A process for preparing trioxane, which comprises

- preparing formaldehyde from methanol by dehydrogenation in a reactor at temperatures in the range from 300 to 1000°C in the presence of a catalyst which is introduced into the reactor with the aid of a carrier gas to give a product gas mixture from which the formaldehyde is separated off and at least part of the remaining product gas mixture is recirculated to the reactor in a circulating gas stream, wherein the carrier gas used is a carbon-free gas or gas mixture, and
- trimerizing the formaldehyde obtained in this way to give trioxane.

**10.** A process for preparing polyoxymethylene, which comprises

- preparing formaldehyde from methanol by dehydrogenation in a reactor at temperatures in the range from 300 to 1000°C in the presence of a catalyst which is introduced into the reactor with the aid of a carrier gas to give a product gas mixture from which the formaldehyde is separated off and at least part of the remaining product gas mixture is recirculated to the reactor in a circulating gas stream, wherein the carrier gas used is a carbon-free gas or gas mixture, and

- if desired, purifying the formaldehyde obtained in this way, and
- polymerizing the formaldehyde.

## Revendications

**1.** Procédé de production de formaldéhyde à partir de méthanol par déshydrogénation dans un réacteur en présence d'un catalyseur, que l'on introduit dans le réacteur à l'aide d'un gaz porteur, à des températures de l'ordre de 300 à 1000°C, avec formation d'un mélange de produits gazeux, **caractérisé en ce que** l'on sépare le formaldéhyde du mélange de produits gazeux et que le mélange de produits gazeux restant est au moins en partie recyclé dans le réacteur dans un courant de gaz de circulation et que l'on utilise comme gaz porteur un gaz ou un mélange de gaz exempts de carbone.

**2.** Procédé selon la revendication 1, **caractérisé en ce que** le gaz exempt de carbone est de l'azote ou de l'hydrogène ou un gaz rare, ou un mélange de ces gaz.

**3.** Procédé selon la revendication 1 ou 2, **caractérisé en ce que** la fraction de gaz porteur exempt de carbone est, par suite de l'amenée de catalyseur, de 1 à 50% du courant total.

**4.** Procédé selon une ou plusieurs des revendications 1 à 3, **caractérisé en ce que** l'on utilise du sodium en tant que catalyseur.

**5.** Procédé selon une ou plusieurs des revendications 1 à 4, **caractérisé en ce que** l'on met en oeuvre en tant que catalyseur des alcoolates de sodium provenant d'alcools inférieurs en $C_1$ à $C_6$.

**6.** Procédé selon une ou plusieurs des revendications 1 à 5, **caractérisé en ce que** l'on met en oeuvre en tant que réacteur un réacteur tubulaire chauffé de l'extérieur.

**7.** Procédé selon une ou plusieurs des revendications 1 à 6, **caractérisé en ce qu'**une partie des sous-produits de la déshydrogénation est utilisée pour la production d'énergie ou comme combustible pour le chauffage du réacteur.

**8.** Procédé selon une ou plusieurs des revendications 1 à 7, **caractérisé en ce que** l'hydrogène libéré en tant que sous-produit est séparé et envoyé à une exploitation.

**9.** Procédé de production de trioxanne, **caractérisé en ce que** l'on produit du formaldéhyde à partir de méthanol par déshydrogénation dans un réacteur en présence d'un catalyseur, que l'on introduit dans le réacteur à l'aide d'un gaz porteur, à des températures de l'ordre de 300 à 1000°C, avec formation d'un mélange de produits gazeux, duquel on sépare le formaldéhyde, le mélange de produits gazeux restant étant au moins en partie recyclé dans le réacteur dans un courant de gaz de circulation, et que l'on utilise comme gaz porteur un gaz ou un mélange de gaz exempts de carbone, et que le formaldéhyde ainsi obtenu est trimérisé en trioxanne.

**10.** Procédé de production de polyoxyméthylène, **caractérisé en ce que**

- on produit du formaldéhyde à partir de méthanol par déshydrogénation dans un réacteur en présence d'un catalyseur, que l'on introduit dans le réacteur à l'aide d'un gaz porteur, à des températures de l'ordre de 300 à 1000°C, avec formation d'un mélange de produits gazeux, duquel on sépare le formaldéhyde, le mélange de produits gazeux restant étant au moins en partie recyclé dans le réacteur dans un courant de gaz de circulation, et l'on utilise comme gaz porteur un gaz ou un mélange de gaz exempts de carbone et
- le formaldéhyde ainsi obtenu est éventuellement purifié, et
- le formaldéhyde est polymérisé.

Fig. 1

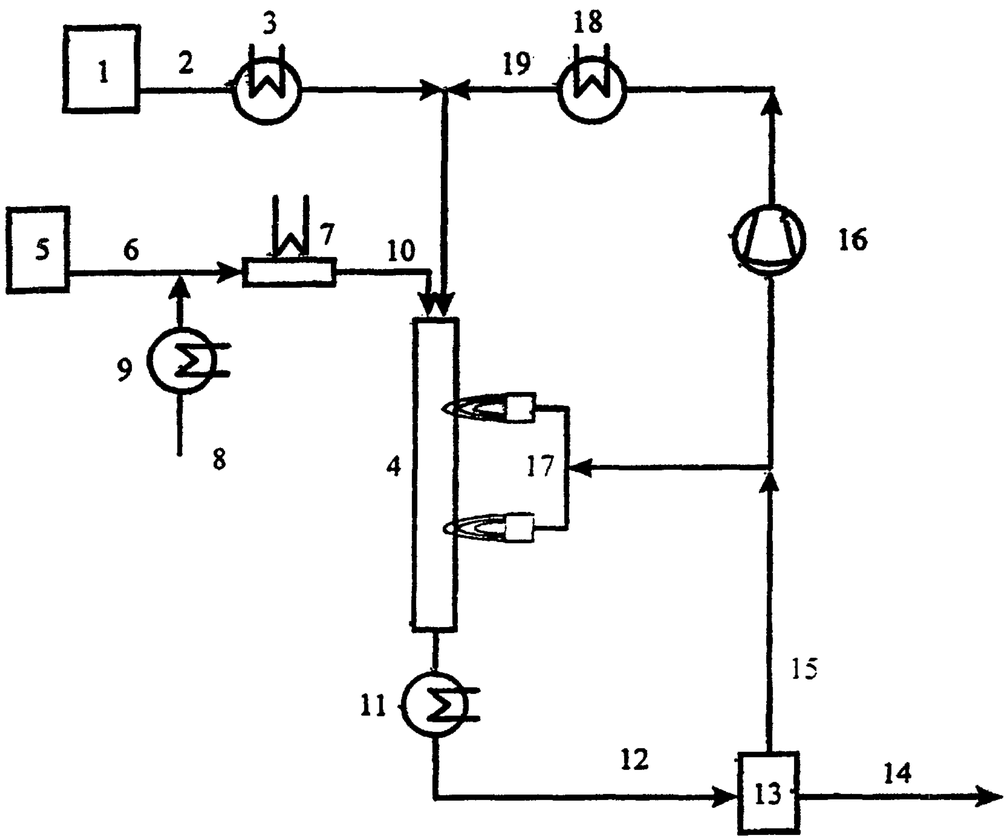

**Fig. 2**

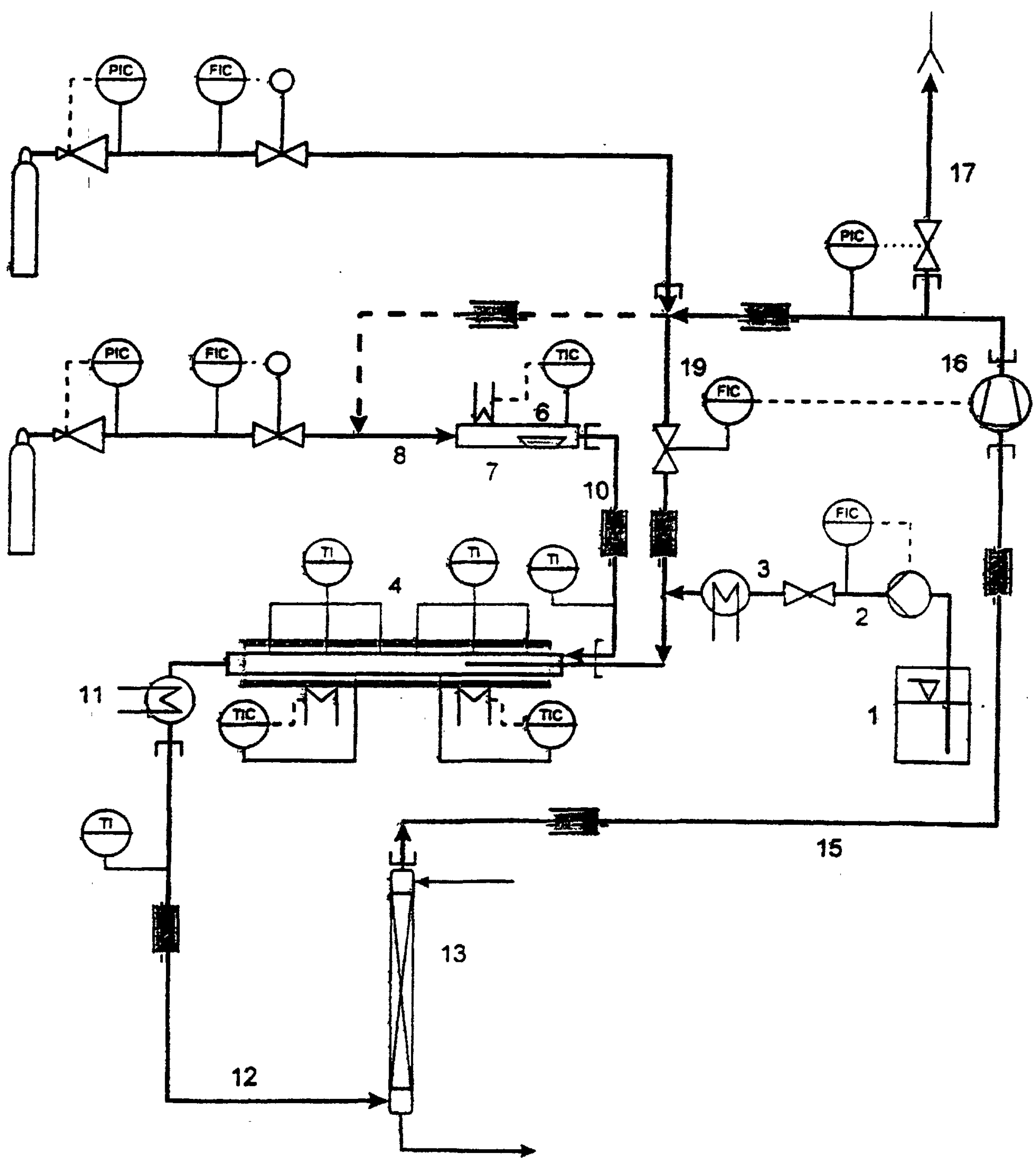
PIC
FIC
17
PIC
19
16
PIC
FIC
TIC
6
FIC
8
7
10
FIC
TI
TI
TI
4
3
2
11
TIC
TIC
1
TI
15
13
12